# EUROPEAN PATENT APPLICATION

(11) **EP 4 098 266 A1**
(43) Date of publication of application: **07.12.2022**
(21) Application number: 20912688.7
(22) Date of filing: 26.11.2020
(51) Int. Cl.: A61K 35/28, A61K 35/12, A61K 38/18, A61K 45/00, A61P 25/00, A61P 43/00

(54) **PHYSICAL FUNCTION RECOVERY PROMOTER**

(30) Priority: 08.01.2020 JP 2020001657
(71) Applicant: Foundation for Biomedical Research and Innovation at Kobe, Kobe-shi, Hyogo 650-0047 (JP)
(72) Inventor: TAGUCHI Akihiko, Kobe-shi, Hyogo 650-0047 (JP); OGAWA Yuko, Kobe-shi, Hyogo 650-0047 (JP); OKINAKA Yuka, Kobe-shi, Hyogo 650-0047 (JP); FUKUSHIMA Masanori, Kobe-shi, Hyogo 650-0047 (JP); YAMANAKA Atsuo, Kobe-shi, Hyogo 650-0047 (JP)
(74) Representative: Germain Maureau
(86) International application number: PCT/JP2020/044082
(87) International publication number: WO 2021/140773

(57) **Abstract**

The effect of physical exercise performed on a physical function of a subject with an irreversibly reduced physical function is promoted. Bone marrow mononuclear cells, CD34-positive cells, CD133-positive cells, or stem cells are contained. The central and peripheral nerves communicate intimately with each other. When these cells are administered to a subject, and the subject performs an appropriate physical exercise, the reduced physical function can be dramatically improved. The physical exercise is, for example, physical exercise that stimulates both of brain and peripheral nerves based on the interactive biofeedback theory. Thus, for example, when bone marrow mononuclear cells are administered to a patient with a sequela of cerebral infarction, and the patient performs an appropriate physical exercise, the effect of functional recovery from the sequela of the cerebral infarction is promoted.

## Description

### TECHNICAL FIELD

The present disclosure relates to a physical function recovery promotor that promotes the effect of physical exercise that is applied to a physical function of a subject that is irreversibly reduced, so as to stimulate both of brain and peripheral nerves in a manner that allows the brain and peripheral nerves to coordinate with each other.

### BACKGROUND ART

The central nerve and the peripheral nerve do not act alone, and communicate intimately with each other and affect each other even during malfunction. For example, in the patient with spinal cord injury, when a brain neuron sends a signal which causes a lower limb to move, the brain neuron does not receive feedback indicating movement of the lower limb, and therefore, learns that the "lower limb does not move." Meanwhile, a peripheral nerve does not receive a stimulus from the brain, and therefore, learns "not to need to move the lower limb." Therefore, mutual adverse effects are added up and made permanent, so that a physical function is irreversibly reduced, leading to a sequela (a residual syndrome or scar such as a functional disorder after an acute syndrome such as a disease or injury has been healed). Thus, such a sequela of spinal cord injury is considered to be a condition resulting from a lack of interactive biofeedback mechanism. The interactive biofeedback refers to "bidirectional biological feedback" between the human brain and nervous systems and the musculoskeletal and peripheral nervous systems. Concerning sequelae of spinal cord injury, it is becoming clarified that the nervous function of the patient is ameliorated by attempting to cause impaired interactive biofeedback to work. For example, in a cybernic treatment employing an electronic attachment called a hybrid assistive limb (HAL), the HAL detects very small neural activity from brain neurons, which is related to a movement of a lower limb, and mechanically moves the lower limb. When a spinal cord injury patient suffering from a sequela uses the HAL, their brain neurons learn that the "lower limb can move," and their peripheral nerves learn to "need to move the lower limb," although the HAL is almost not used in the acute phase of spinal cord injury. This promotes interactive biofeedback between the central nerve and the peripheral nerve, leading to restoration of the nervous function. The HAL for medical use - lower limb type (medical HAL) was developed as a biological signal-activated motor function-improving device and has been approved as a medical device since November, 2015. It was demonstrated that a walking movement treatment using the HAL for medical use - lower limb type has the effect of improving the movement ability of a patient with ALS, a muscular dystrophy, or the like by about 24%, and the treatment based on the interactive biofeedback theory is effective in treating nervous and muscular diseases. However, physical exercise based on interactive biofeedback, when performed alone, is not enough to restore the nervous function, and therefore, there is a demand for a combination therapy that further improves and sustains the nervous function restoration effect of such physical exercise. Among the potential drugs that promote the nervous function restoration effect of physical exercise based on the interactive biofeedback theory is, for example, nusinersen. A nervous function restoration effect of the combination of physical exercise based on the interactive biofeedback theory with nusinersen is expected.

In the treatment of sequelae of cerebral infarction, physical exercise based on the interactive biofeedback theory is expected to provide nervous function restoration. A typical treatment for sequelae of cerebral infarction which is called functional maintenance rehabilitation is conducted in order to prevent a reduction in functions and thereby maintain and continue home life and social life. Thus, the goal of functional maintenance rehabilitation is to maintain the current state of an already reduced nervous function, and therefore, attention is focused only on movement of the body. Thus, functional maintenance rehabilitation is not based on the interactive biofeedback theory, which promotes enhancement of neurotransmission involved in movement of the body.

It has been demonstrated that cell therapy employing hematopoietic stem cells is effective in regeneration of nervous tissue impaired by acute cerebral infarction. NON-PATENT DOCUMENT 1 describes a treatment for acute cerebral infarction in which autologous bone marrow-derived mononuclear cells are intravenously administered to a patient. NON-PATENT DOCUMENT 1 describes a treatment that is directed to a group of patients with severe cardiogenic brain embolism syndromes and having insufficient nervous function restoration within 10 days after the onset of cerebral infarction, and in which bone marrow cells are collected under local anesthesia, and a mononuclear cell fraction is separated by specific gravity centrifugation, and is intravenously administered to the patient. However, according to a study of timing of a treatment by administering hematopoietic stem cells, administration of hematopoietic stem cells is effective in treating acute and subacute phases on day 2, 4, 7, 10, and 14 after cerebral infarction, and is not effective in treating sequelae of cerebral infarction (NON-PATENT DOCUMENT 2). NON-PATENT DOCUMENT 3 describes cell therapy in which hematopoietic stem cells are administered to cerebral infarction patients 18 days (average) after the onset of cerebral infarction, and indicates that although it was conducted in combination with typical rehabilitation, the treatment was not effective at all. Thus, such a treatment method for restoring a function impaired by cerebral infarction is limited to the acute phase, and there is no treatment method for sequelae. Therefore, there is a demand for a physical function recovery promotor that promotes the nervous function restoration effect of physical exercise based on the interactive biofeedback theory for sequelae of cerebral infarction patients.

### CITATION LIST

### NON-PATENT DOCUMENTS

NON-PATENT DOCUMENT 1: Taguchi A et al. Intravenous Autologous Bone Marrow Mononuclear Cell Transplantation for Stroke: Phase1/2a Clinical Trial in a Homogeneous Group of Stroke Patients. Stem Cells Dev. 2015 Oct 1;24(19):2207-18
NON-PATENT DOCUMENT 2: Uemura M, et al. Cell-based therapy to promote angiogenesis in the brain following ischemic damage. Curr Vasc Pharmacol. 2012 May;10(3):285-8.
NON-PATENT DOCUMENT 3: Prasad K et al. Intravenous autologous bone marrow mononuclear stem cell therapy for ischemic stroke: a multicentric, randomized trial. Stroke. 2014 Dec;45(12):3618-24.

### SUMMARY OF THE INVENTION

### TECHNICAL PROBLEM

With the above circumstances in mind, the present invention has been made. It is an object of the present invention to provide a physical function recovery promotor that promotes an effect of physical exercise which is applied to an irreversibly reduced physical function of a subject so as to stimulate both of brain and peripheral nerves.

### SOLUTION TO THE PROBLEM

A physical function recovery promotor according to the present invention is for promoting an effect of physical exercise that allows central and peripheral nerves to coordinate with each other in order to restore a reduced physical function of a subject, the physical function including a motor function, a cognitive function, and/or a sensory function, the promotor having bone marrow mononuclear cells, CD34-positive cells, CD133-positive cells, or stem cells.

A physical function recovery promotor according to the present invention is for promoting an effect of physical exercise that allows central and peripheral nerves to coordinate with each other in order to restore a reduced physical function of a subject caused by a sequela of cerebral infarction, the physical function including a motor function, a cognitive function, and/or a sensory function, the promotor having bone marrow mononuclear cells.

A physical function recovery promotor according to the present invention is for promoting an effect of physical exercise for restore a reduced physical function of a subject caused by a sequela of cerebral infarction, the physical function including a motor function, a cognitive function, and/or a sensory function, the promotor having CD34-positive cells.

### BRIEF DESCRIPTION OF THE DRAWINGS

[FIG. 1] FIG. 1 shows the result of a passive avoidance test in the case where bone marrow mononuclear cells are administered.
[FIG. 2] FIG. 2 shows the result of a wire hang test in the case where bone marrow mononuclear cells are administered.
[FIG. 3] FIG. 3 shows the result of a wire hang test in the case where CD34-positive cells are administered.
[FIG. 4] FIG. 4 shows the result of a water maze test in the case where bone marrow mononuclear cells are administered.
[FIG. 5] FIG. 5 shows the result of a water maze test in the case where CD34-positive cells are administered.
[FIG. 6] FIG. 6 shows the result of a rotor-rod test in the case where bone marrow mononuclear cells are administered.

### DESCRIPTION OF EMBODIMENTS

Embodiments of the present invention will be specifically described below with reference to the accompanying drawings. The embodiments are for the purpose of facilitating understanding of the principle of the present invention. The scope of the present invention is not intended to be limited to the embodiments below. Those skilled in the art will make substitutions to the embodiments when necessary without departing from the scope of the present invention.

A physical function recovery promotor according to the present invention has bone marrow mononuclear cells, CD34-positive cells, CD133-positive cells, or stem cells, and is administered to a subject having a reduced physical function before or after start of physical exercise for restoring the physical function of the subject. The physical function recovery promotor of the present invention is a cell formulation having bone marrow mononuclear cells, CD34-positive cells, CD133-positive cells, or stem cells, and is provided to address the problem of promoting an effect of physical exercise for allowing central and peripheral nerves to coordinate with each other in order to restore a reduced physical function of a subject. The problem of providing such a cell formulation is novel. The way to use the cell formulation of the present invention, i.e., administering the cell formulation of the present invention before or after start of physical exercise, is novel. The cells used in the cell formulation of the present invention may be either isolated cells or cultured cells.

The physical function includes a motor function, a cognitive function, and/or a sensory function.

The motor function includes human functions relating to muscle strength, endurance, agility, balance, speed, and/or flexibility.

The cognitive function includes human functions relating to understanding, decision, calculation, thinking, orientation, memory, and/or learning.

The sensory function includes human factions relating to sense of vision, sense of hearing, sense of smell, sense of taste, and/or sense of touch.

A physical function recovery promotion method according to the present invention is characterized by having: (i) administering any of bone marrow mononuclear cells, CD34-positive cells, CD133-positive cells, or stem cells before start of physical exercise for allowing central and peripheral nerves to coordinate with each other, and (ii) causing the physical exercise for allowing central and peripheral nerves to coordinate with each other to be performed.

There is a linkage between central and peripheral nerves. The present inventors have found that when a subject having a reduced physical function performs physical exercise for restoring the physical function, then if bone marrow mononuclear cells, CD34-positive cells, CD133-positive cells, or stem cells are administered to the subject, the physical function is dramatically improved and maintained, and based on this novel finding, have completed the present invention. When a subject having a reduced physical function performs physical exercise based on the interactive biofeedback theory, then if bone marrow mononuclear cells, CD34-positive cells, CD133-positive cells, or stem cells are administered to the subject, the effect of physical exercise based on the interactive biofeedback theory can also be dramatically improved.

In the present invention, bone marrow mononuclear cells are preferably intravenously administered, but the present invention is not limited to this. CD34-positive cells and CD133-positive cells are preferably administered into a common carotid artery, internal carotid artery, anterior cerebral artery, middle cerebral artery, posterior cerebral artery, or vertebral artery, particularly preferably a common carotid artery or vertebral artery, but the present invention is not limited to this.

The CD34-positive cells or CD133-positive cells administered to the subject may be, but not limited to, umbilical cord blood-derived cells, human bone marrow-derived cells, human peripheral blood-derived cells, fetal liver-derived cells, or the like, preferably human umbilical cord blood-derived cells or human peripheral blood-derived cells.

The concentration of the bone marrow mononuclear cells, CD34-positive cells, CD133-positive cells, or stem cells administered to the subject may, for example, be, but not limited to, 5 × 10⁴/kg to 1 × 10⁷/kg, preferably 1 × 10⁵/kg to 1 × 10⁶/kg, particularly preferably 5 × 10⁵/kg. For example, when bone marrow mononuclear cells are intravenously administered, the concentration thereof is preferably 5 × 10⁶/kg. When CD34-positive cells are intraarterially administered, the concentration thereof is preferably 5 × 10⁵/kg. When CD133-positive cells are intraarterially administered, the concentration thereof is preferably 5 × 10⁵/kg. When umbilical cord blood mononuclear cells are intraarterially administered, the concentration thereof is preferably 5 × 10⁶/kg.

Examples of the stem cells include, but are not limited to, ES cells, EC cells, EG cells, iPS cells, hematopoietic stem cells, mesenchymal stem cells, hepatic stem cells, splenic stem cells, skin stem cells, umbilical cord blood stem cells, bone marrow stem cells, muscle stem cells, germ stem cells, fat stem cells, dental pulp stem cells, and MUSE cells.

The physical exercise performed by the subject in order to restore a physical function is a physical activity that is performed in a planned and intentional way in order to improve physical strength and that can be continued, preferably physical exercise based on the interactive biofeedback theory.

The reduced physical function of the subject is caused by, for example, an ischemic disease.

The ischemic disease is, but not limited to, any disease selected from myocardial infarction, unstable angina, graft occlusion after coronary artery bypass surgery, coronary artery occlusion after percutaneous transluminal coronary angioplasty, vascular occlusion after revascularization, arteriosclerosis obliterans, thromboangiitis obliterans, essential thrombocythemia, thrombotic thrombocytopenic purpura, antiphospholipid antibody syndrome, and Kawasaki disease. The ischemic disease is, for example, cerebral palsy. Cerebral palsy is caused by, for example, neonatal hypoxic-ischemic encephalopathy, bilirubin encephalopathy in preterm infants, or the like. The ischemic disease is preferably a sequela of cerebral infarction. In the case of cerebral infarction, it is considered that the symptoms of cerebral infarction become stationary after, for example, 6 months from the onset, and a nervous function disorder remaining after, for example, 6 months have passed since the onset is defined as a sequela of cerebral infarction. Meanwhile, it is considered that, for example, 4 weeks after production of a cerebral infarction model of a mouse is equivalent to 6 months after the onset of cerebral infarction of a human. After this period of time, the symptoms have become substantially stationary, maintenance rehabilitation is performed, and relapse prophylaxis is centered, and cell necrosis or the occurrence of a scar in tissue is observed. At that time, even when hematopoietic stem cells are administered in order to treat a sequela treatment of cerebral infarction, the treatment effect of the cell administration alone is low. Rehabilitation performed for a sequela of cerebral infarction is for maintaining a function, and cannot be expected to restore an impaired function. However, if bone marrow mononuclear cells, CD34-positive cells, CD133-positive cells, or stem cells are administered to a patient having a sequela of cerebral infarction, the effect of physical exercise that allows peripheral and central nerves to coordinate with each other on a sequela of cerebral infarction is promoted. The administered cells are preferably bone marrow mononuclear cells or CD34-positive cells. Bone marrow mononuclear cells are preferably intravenously administered. CD34-positive cells are preferably administered into a common carotid artery or internal carotid artery.

The physical function recovery promotor of the present invention is administered to a subject for treatment of a sequela of cerebral infarction before, during, or after start of physical exercise. The timing of administration is, for example, within a period of time between any two selected from time points 6 weeks before, 4 weeks before, 2 weeks before, 1 week before, 3 days before, during start, 3 days after, 1 week after, or 2 weeks after start of physical exercise, preferably a period of time between 4 weeks before start of physical exercise and the time to start physical exercise.

The cerebral infarction to be treated is preferably lacunar infarction, atherothrombosis cerebral infarction, or cardiogenic brain embolism.

The reduced physical function of the subject is caused by, for example, an intractable nervous disease.

Examples of the intractable nervous disease include, but are not limited to, amyotrophic lateral sclerosis (ALS), Parkinson's disease, multiple system atrophy, spinocerebellar ataxia, progressive supranuclear palsy, multiple sclerosis, syringomyelia, Guillain-Barre syndrome, spinal muscular atrophy, and myasthenia gravis.

The reduced physical function of the subject is also caused by, for example, dementia.

Examples of the dementia include, but are not limited to, Alzheimer's disease dementia, dementia with Lewy bodies, frontotemporal dementia, corticobasal degeneration, cerebrovascular dementia, Down syndrome, and Huntington's disease.

The reduced physical function of the subject is also caused by, for example, autism.

The reduced physical function of the subject is also caused by, for example, developmental disability or behavioral disorder.

The reduced physical function of the subject is also caused by, for example, spinal cord injury.

The reduced physical function of the subject is caused not only by disease but also by aging when the subject is an older person. The present invention is also applied in order to improve a reduced physical function caused by aging. Although not restricted, an older person is defined as a person aged at least 60, preferably at least 70, and more preferably 75 to 95.

In the present invention, the physical function recovery promotor can further contain a neurotrophin. Examples of the neurotrophin include, but are not limited to, VEGFs, angiopoietins, PDGFs, TGF-βs, FGFs, PIGFs, matrix metalloproteinases, and plasminogen activators. Note that it is desirable to avoid administering a drug that has the effect of releasing hematopoietic stem cells into peripheral blood (granulocyte-colony stimulating factor (G-CSF)). This is because administration of G-CSF may lead to brain shrinkage or a reduction in a nervous function caused by release of granulocytes from bone marrow.

When the physical function recovery promotor of the present invention is used in the form of an injection preparation, additives that are commonly used in the art can be used if necessary. Examples of the additive include tonicity agents, stabilizing agents, buffering agents, preserving agents, chelating agents, antioxidants, and solubilizing agents. Examples of the tonicity agents include sugars, such as glucose, sorbitol, and mannitol, sodium chloride, glycerol, propylene glycol, and polyethylene glycol. Examples of the stabilizing agents include sodium sulfite. Examples of the buffering agents include boric acid buffers, phosphoric acid buffers, citric acid buffers, tartaric acid buffers, and acetic acid buffers. Examples of the preserving agents include paraoxybenzoic acid esters, benzyl alcohol, chlorocresol, phenethyl alcohol, and benzethonium chloride. Examples of the chelating agents include disodium EDTA and sodium citrate. Examples of the antioxidants include sodium sulfite, sodium hydrogen sulfite, sodium ascorbate, and sodium thiosulfate. Examples of the solubilizing agents include dextran, polyvinyl pyrrolidone, sodium benzoate, ethylenediamine, salicylamide, nicotinamide, and polyoxyethylene hydrogenated castor oil derivatives.

### Examples

### 1-1. Production of cerebral infarction sequela model mice

A cerebral infarction model (JP 4481706 B2) was produced using the following procedure. A severe combined immunodeficiency mouse (SCID mouse; C.B-17/Icr-scid/IcidJcl) aged 7 weeks was fully anesthetized with isoflurane, and the skull base was drilled by about 1.5 mm so that the left middle cerebral artery could be directly reached by approaching from the left zygomatic region. The left middle cerebral artery immediately after passing through the olfactory tract (distal side of the olfactory tract crossing section) was coagulated using a bipolar electrocautery and cut after coagulation, thereby permanently occluding the left middle cerebral artery. Thus, a cerebral infarction model with the affected portion localized to the cortex of the left middle cerebral artery region was prepared. In this model mouse, a nervous function impairment remained even during a period of time corresponding to a chronic phase 4 weeks after the production of cerebral infarction. Therefore, this model mouse was used as a cerebral infarction sequela model mouse. Note that after the production of cerebral infarction, five model mice were kept in each cage and maintained in a state that the mice constantly received slight nerve stimuli from each other. Thus, the model mice were kept under conditions that are considered to prevent disuse muscle atrophy or the like so that all the mice were subjected to a general functional maintenance rehabilitation treatment.

### 1-2. Preparation of cells to be administered

Cells to be administered were prepared in the following procedure. Bone marrow mononuclear cells were prepared as follows: bone marrow fluid was collected from a mouse femur and tibia, a bone marrow cell suspension was laid on top of Ficoll solution, which is a specific gravity centrifugation liquid, and a swing rotor centrifuge was used to perform centrifugation at 600 g for 20 minutes; and a mononuclear cell fraction observed immediately above the specific gravity centrifugation liquid layer was collected with a pipette. CD34-positive cells were prepared as follows: GCSF-mobilized human peripheral blood was purchased from Stemexpress, and CD34-positive cells were isolated using the CliniMACS System.

### 1-3. Administration of cells

Concerning bone marrow mononuclear cells, 1 × 10⁵ mouse-derived bone marrow mononuclear cells were administered to the above-described cerebral infarction sequela model mice through their tail veins (cell therapy group, six mice). As a comparative example, cerebral infarction sequela model mice to which PBS was administered through their tail veins were used (PBS group, six mice). Concerning CD34-positive cells, 1 × 10⁴ human peripheral blood CD34-positive cells were administered to cerebral infarction sequela model mice through their carotid arteries (cell therapy group, nine mice). As a comparative example, cerebral infarction sequela model mice to which PBS was administered through their tail veins were used (PBS group, eight mice).

### 1-4. Physical exercise stimulating both of brain and peripheral nerves, and cerebral and nervous function test

In a cerebral and nervous function test, a cell therapy group of cerebral infarction sequela model mice to which bone marrow cells were intravenously administered, a PBS group of cerebral infarction sequela model mice to which PBS was intravenously administered, and a non-treated group (no-surgery group, six mice) were compared. The HAL electronic attachment for implementing the interactive biofeedback theory is present only for humans, and no such an attachment is not present for mice. Therefore, physical exercise that stimulates both of brain and peripheral nerves in a manner that allows the brain and peripheral nerves to coordinate with each other was used.

### 1-4-1. Passive avoidance test

A passive avoidance test was conducted as a cerebral and nervous function test. In the passive avoidance test, a modified version of the TMS-2 device, manufactured by Melquest Ltd., was used as a passive avoidance experiment device. In the device, a light compartment and a dark compartment were linked together. The light and dark compartments had the same size, i.e., 120 (w) × 120 (D) × 135 (H). In the passive avoidance test using the device, when an animal entered from the light compartment to the dark compartment, an electrical stimulus was applied to the animal so as to cause the animal to learn an association between entrance to the dark compartment and the fear of shock. A mouse was placed in the light compartment of the device, and 10 seconds after that, the door was opened to allow the mouse to move into the dark compartment. After the mouse entered the dark compartment, the door was closed, and 10 seconds after that, an electrical stimulus (20 mA, 3 seconds) was applied to the mouse. The mouse was caused to learn an association between entrance to the dark compartment and the fear of a pain caused by the electrical stimulus, and learn the unfavorable stimulus (electrical stimulus). 24 hours later, the mouse was placed in the light compartment again, and the time (seconds) for which the mouse remained in the light compartment was measured.

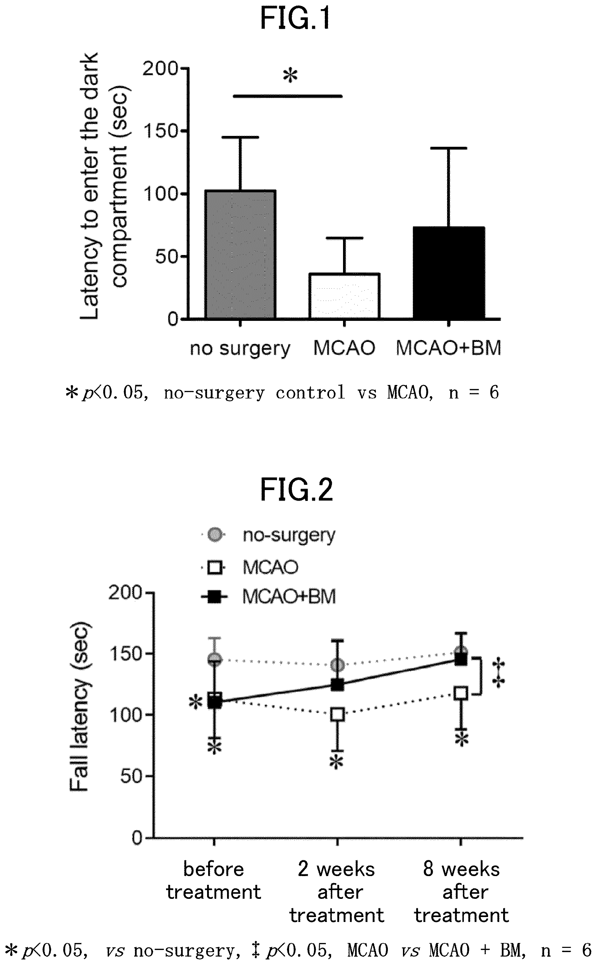

As shown in FIG. 1, the average time that the no-surgery mouse remained in the light compartment was 100 seconds. PBS was intravenously administered to cerebral infarction sequela model mice 4 weeks after production of cerebral infarction. Thereafter, the mice were not subjected to physical exercise that stimulates both of brain and peripheral nerves. Scoring was conducted 2 weeks after intravenous administration, which is described below in the Wire hang test section and the Water maze test section. The average score was 38 seconds (denoted by MCAO in FIG. 1). A significant decrease in learning ability was observed in the PBS group compared to the no-surgery group. Bone marrow mononuclear cells were intravenously administered to cerebral infarction sequela model mice 4 weeks after production of cerebral infarction, and thereafter, the mice was not subjected to test exercise. Scoring was conducted 2 weeks after intravenous administration, and the average score was 75 seconds (denoted by MCAO + BM in FIG. 1). There was no statistically significant difference between the cell-administered group and the PBS group.

The above result demonstrated that there is no significant difference between the score of cerebral infarction sequela model mice 4 weeks after production of cerebral infarction as measured 2 weeks after intravenous administration of bone marrow mononuclear cells, and the score of cerebral infarction sequela model mice as measured 2 weeks after intravenous administration of PBS. Thus, it was demonstrated that when bone marrow mononuclear cells are administered to a cerebral infarction sequela model mouse, then if the cerebral infarction sequela model mouse is not subjected to physical exercise that stimulates both of brain and peripheral nerves, the cerebral and nervous function of the cerebral infarction sequela model mouse is not improved.

### 1-4-2. Wire hang test

A wire hang test was conducted as a cerebral and nervous function test. In the wire hang test, a mouse was placed at the center of a 30 cm × 30 cm wire screen with a mesh size of 1 cm. The wire screen was inverted, and suspended on a support disposed at a height of about 40 cm above an open cage with deep bedding. The latency to when a mouse falls from the wire screen was measured. A mouse that did not fall throughout the test period was given a latency of 180 seconds. In the test, a stimulus relating the fear of fall was provided to the brain, and was strongly associated with clinging movement to the wire screen, whereby both of brain and peripheral nerves were simultaneously stimulated.

PBS was intravenously administered to cerebral infarction sequela model mice 4 weeks after production of cerebral infarction, and thereafter, test exercise was continued. Scoring was conducted 2 and 8 weeks after intravenous administration (denoted by MCAO in FIG. 2). Bone marrow mononuclear cells were intravenously administered to cerebral infarction sequela model mice 4 weeks after production of cerebral infarction, and thereafter, test exercise was continued. Scoring was conducted 2 and 8 weeks after intravenous administration (denoted by MCAO + BM in FIG. 2).

As shown in FIG. 2, before administration, there was a significant difference between both of the cell therapy group and the PBS-administered group, and the no-surgery group. It was observed that the PBS group had a significant reduction in motor function 2 weeks after intravenous administration compared to the no-surgery group. However, it was observed that the cell-administered group had a motor function restoration compared to the PBS group. The result of the third test conducted 8 weeks after intravenous administration showed that the cell-administered group had a function restoration to the same level of the no-surgery group, and there was a statistically significant difference between the cell-administered group and the PBS group. Meanwhile, it was observed that the PBS group had a significant reduction in motor function compared to the no-surgery group.

The above result demonstrated that for the group of cerebral infarction sequela model mice to which bone marrow mononuclear cells have been intravenously administered 4 weeks after production of cerebral infarction, the repetition of physical exercise that stimulates both of brain and peripheral nerves in a manner that allows the brain and peripheral nerves to coordinate with each other is effective in improving a cerebral and nervous function thereof.

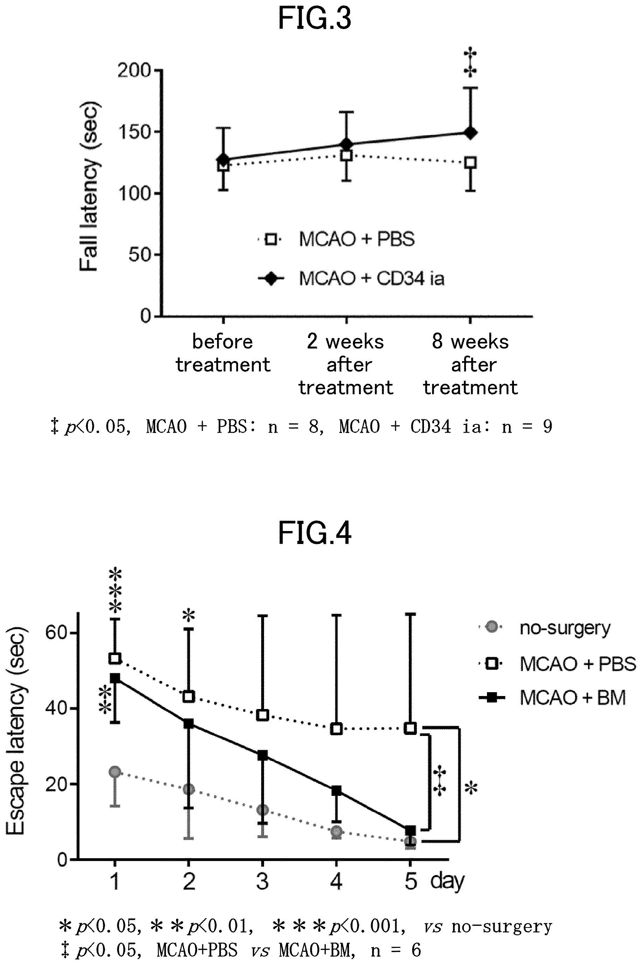

CD34-positive cells were intraarterially administered to cerebral infarction sequela model mice 4 weeks after production of cerebral infarction, and thereafter, test exercise was continued. Scoring was conducted 2 and 8 weeks after administration. The mice were compared with the group of cerebral infarction sequela model mice to which PBS was administered 4 weeks after production of cerebral infarction (in FIG. 3, the PBS-administered group is denoted by MCAO + PBS, and the CD34-positive cell-administered group is denoted by MCAO + CD34 ia). As shown in FIG. 3, 2 weeks after intraarterial administration, no significant difference was observed between the CD34-positive cell-administered group and the PBS group, and an increase in score was observed. Eight weeks after administration, a significant difference was observed between the CD34-positive cell-administered group and the PBS group.

When the level at 8 weeks after administration was compared with that before administration to measure a change amount, the average value of the CD34-positive cell-administered group was 33.16, and the average value of the PBS group was 2.28. Thus, for the CD34-positive cell-administered group, there was a significant increase from the level before administration (p = 0.0136).

The above result demonstrated that the group of cerebral infarction sequela model mice to which CD34-positive cells have been intraarterially administered 4 weeks after production of cerebral infarction, the repetition of physical exercise that stimulates both of brain and peripheral nerves in a manner that allows the brain and peripheral nerves to coordinate with each other is effective in improving a cerebral and nervous function thereof.

### 1-4-3. Water maze test

### Test 1

A water maze test was conducted as a cerebral and nervous function test. In the water maze test, a circular pool was filled with water, and a platform was submerged about 1 cm below the water's surface. Each mouse was allowed to find and mount the escape platform. The time (seconds) it took for the mouse to reach the escape platform was measured. In the test, a stimulus related to the fear of drowning was applied to the brain so that spatial cognition and swimming exercise were strongly associated with each other, whereby both of brain and peripheral nerves were simultaneously stimulated.

As shown in FIG. 4, PBS was intravenously administered to cerebral infarction sequela model mice 4 weeks after production of cerebral infarction, and thereafter, the mice were not subjected to physical exercise that stimulates both of brain and peripheral nerves. Scoring was conducted 4 weeks after intravenous administration. As a result, the average score of the PBS group was 53 seconds on Day 1 (before physical exercise) (denoted by MCAO + PBS in FIG. 4). Bone marrow mononuclear cells were intravenously administered to cerebral infarction sequela model mice 4 weeks after production of cerebral infarction, and thereafter, the mice were not subjected to physical exercise that stimulates both of brain and peripheral nerves. Scoring was conducted 4 weeks after intravenous administration. As a result, the average score of the cell therapy group was 49 seconds on Day 1 (before physical exercise) (denoted by MCAO + BM in FIG. 4). The average value of the no-surgery group was 22 seconds on Day 1 (before physical exercise) (denoted by no-surgery in FIG. 4).

The result of statistical analysis showed that the p-value of the difference between the cell therapy group and the PBS group was 0.65, which is not a significant difference. The p-value of the difference between the cell therapy group and the no-surgery group was 0.002, which is a significant difference. The p-value of the difference between the PBS group and the no-surgery group was 0.0004, which is a significant difference. Thus, it was demonstrated that even when bone marrow mononuclear cells are administered, then if physical exercise that stimulates both of brain and peripheral nerves is absent, the effect of improving a cerebral and nervous function against a sequela of cerebral infarction is not obtained.

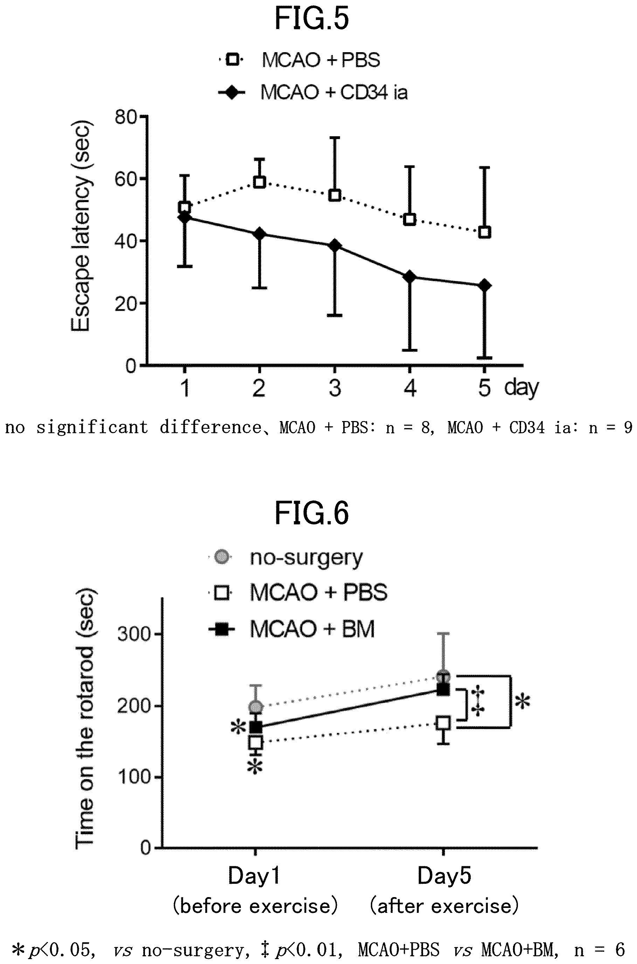

PBS was intraarterially administered to cerebral infarction sequela model mice 4 weeks after production of cerebral infarction, and thereafter, the mice were not subjected to test exercise. Scoring was conducted 4 weeks after intraarterial administration (FIG. 5). As a result, the average score of the PBS group was 50.9 seconds on Day 1 (before physical exercise) (denoted by MCAO + PBS in FIG. 5). CD34-positive cells were intraarterially administered to cerebral infarction sequela model mice 4 weeks after production of cerebral infarction. Scoring was conducted 4 weeks after intraarterial administration. As a result, the average score of the cell therapy group was 47.6 seconds on Day 1 (before physical exercise) (denoted by MCAO + CD34 ia in FIG. 5).

The result of statistical analysis showed that there was not a significant difference between the cell therapy group and the PBS group. Thus, it was demonstrated that even when CD34-positive cells are intraarterially administered, then if physical exercise that stimulates both of brain and peripheral nerves is absent, the effect of improving a cerebral and nervous function against a sequela of cerebral infarction is not obtained.

### Test 2

The effect of physical exercise that stimulates both of brain and peripheral nerves on cerebral infarction sequela model mice was studied. Specifically, a water maze test was conducted every day using a water maze test device including a circular pool filled with water, to provide physical exercise for improving motor and memory functions impaired by cerebral infarction, i.e., for stimulating both of brain and peripheral nerves in a manner that allows the brain and peripheral nerves to coordinate with each other, on Days 2, 3, and 4.

The effect of physical exercise that stimulates both of brain and peripheral nerves in a manner that allows the brain and peripheral nerves to coordinate with each other, on mice to which PBS or bone marrow mononuclear cells had been administered, was studied.

The average value of the PBS group was 35 seconds on Day 5 (after exercise). The level on Day 5 was compared with that on Day 1 (before exercise) for statistical analysis to determine whether or not training had any treatment effect. As a result, the p-value was 0.11, which means that there is not a significant difference between before and after training. Thus, it was demonstrated that for the PBS-administered group, physical exercise that stimulates both of brain and peripheral nerves in a manner that allows the brain and peripheral nerves to coordinate with each other, for a sequela of cerebral infarction, is not effective in improving a cerebral and nervous function.

The average value of the bone marrow mononuclear cell group was 8 seconds on Day 5 (after exercise). The level on Day 5 was compared with that on Day 1 (before exercise) for statistical analysis to determine whether or not training had any treatment effect. As a result, the p-value was 0.00025, i.e., a significant reduction between before and after training was observed. Thus, it was demonstrated that for the bone marrow mononuclear cell group, physical exercise that stimulates both of brain and peripheral nerves in a manner that allows the brain and peripheral nerves to coordinate with each other, for a sequela of cerebral infarction, significantly improves and sustains a cerebral and nervous function (FIG. 4).

Next, the groups on Day 5 (after exercise) were compared with each other. As a result, the p-value of the difference between the cell therapy group and the PBS group was 0.04, and thus, a statistically significant improvement in a cerebral and nervous function was observed. The p-value of the difference between the cell therapy group and the no-surgery group was 0.95, and thus, no significant difference was observed. The p-value of the difference between the PBS group and the no-surgery group was 0.02, and thus, a significant difference was observed (FIG. 4).

For the group of cerebral infarction sequela model mice to which PBS had been administered, and the group of cerebral infarction sequela model mice to which bone marrow mononuclear cells had been administered, the effect of physical exercise that stimulates both of brain and peripheral nerves in a manner that allows the brain and peripheral nerves to coordinate with each other was studied.

In the case of CD34-positive cells, the time it took to reach the escape platform tended to gradually become shorter during training by physical exercise that stimulates both of brain and peripheral nerves (FIG. 5). Therefore, it was demonstrated that in the case of intraarterial administration of CD34-positive cells, physical exercise that stimulates both of brain and peripheral nerves of cerebral infarction sequela model mice significantly improves a cerebral and nervous function thereof.

The results of tests 1 and 2 demonstrated that (i) for cerebral infarction sequela model mice to which only bone marrow mononuclear cells or CD34-positive cells have been administered, general physical exercise is not effective in improving a cerebral and nervous function, (ii) for cerebral infarction sequela model mice, physical exercise that stimulates both of brain and peripheral nerves, alone, is not effective in improving a cerebral and nervous function, and (iii) due to administration of bone marrow mononuclear cells or CD34-positive cells to cerebral infarction sequela model mice, a therapeutic effect for a sequela of cerebral infarction emerges from physical exercise that stimulates both of brain and peripheral nerves of the cerebral infarction sequela model mice in a manner that allows the brain and peripheral nerves to coordinate with each other. Thus, it was demonstrated that administration of bone marrow mononuclear cells or CD34-positive cells promotes the effect of physical exercise that stimulates both of brain and peripheral nerves on a sequela of cerebral infarction.

### 1-4-4. Rotor-rod test

### Test 1

The rotor-rod test (rotarod test) was employed as a cerebral and nervous function test. The rotor-rod test is for measuring the motor coordination and balance of a mouse. A device, MK630A (Muromachi Kikai Co., Ltd.), was used. The device was programmed to accelerate a rod to reach 4-40 rpm in 300 seconds. A mouse is placed on the rotating rod of the device. The time (seconds) for which the mouse stays on the rotating rod until the mouse falls was recorded.

As shown in FIG. 6 described below, bone marrow mononuclear cells were intravenously administered to cerebral infarction sequela model mice 4 weeks after production of cerebral infarction. Scoring was conducted 8 weeks after intravenous administration. As a result, the average of the cell therapy group (denoted by MCAO + BM in FIG. 6) was 170 seconds on Day 1 (before exercise). The average of the PBS-administered group (denoted by MCAO + PBS in FIG. 6) was 148 seconds on Day 1 (before exercise). The average of the no-surgery group was 198 seconds on Day 1 (before exercise). There was no statistically significant difference between the cell-administered group and the PBS group. Thus, it was demonstrated that even when bone marrow mononuclear cells are administered, then if physical exercise that stimulates both of brain and peripheral nerves in a manner that allows the brain and peripheral nerves to coordinate with each other is absent, the effect of improving a cerebral and nervous function on a sequela of cerebral infarction is not obtained.

### Test 2

As physical exercise that stimulates both of brain and peripheral nerves in a manner that allows the brain and peripheral nerves to coordinate with each other, for a sequela of cerebral infarction, all of the groups were subjected to motor training using a device for use in the rotor-rod test, on Day 2, Day 3, and Day 4 for 5 minutes each day. Specifically, a mouse was placed on the rotating rod, and after falling from the rotating rod, was returned to the rotating rod. As a result, the fear and anxiety of fall were given to the brain, and were strongly associated with an improvement in motor coordination and balance functions. This physical exercise that stimulates both of brain and peripheral nerves in a manner that allows the brain and peripheral nerves to coordinate with each other was conducted during 3 days.

In the rotor-rod test on Day 5 (after exercise), the average score of the cell therapy group was 223 seconds, and the average score of the PBS group was 176 seconds. The result of statistical analysis showed that the p-value of the difference therebetween was 0.01, which is a significant difference.

The results of Tests 1 and 2 demonstrated that (i) for a sequela of cerebral infarction, administration of bone marrow mononuclear cells or general physical exercise, alone, is not effective in improving a cerebral and nervous function, and (ii) due to administration of bone marrow mononuclear cells, a therapeutic effect for a sequela of cerebral infarction emerges from exercise that stimulates both of brain and peripheral nerves in a manner that allows the brain and peripheral nerves to coordinate with each other. Thus, it was demonstrated that administration of bone marrow mononuclear cells promotes the effect of exercise that simultaneously stimulates both of brain and peripheral nerves on a sequela of cerebral infarction.

Consequently, it was demonstrated that administration of bone marrow mononuclear cells promotes the effect of exercise that simultaneously stimulates both of brain and peripheral nerves on a sequela of cerebral infarction.

### INDUSTRIAL APPLICABILITY

The embodiments are useful in a treatment that promotes the effect of physical exercise that stimulates both of brain and peripheral nerves so as to allow the brain and peripheral nerves to coordinate with each other.

## Claims

1. A physical function recovery promotor for promoting an effect of physical exercise that allows central and peripheral nerves to coordinate with each other in order to restore a reduced physical function of a subject, the physical function including a motor function, a cognitive function, and/or a sensory function, the promotor comprising:
bone marrow mononuclear cells, CD34-positive cells, CD133-positive cells, or stem cells.

2. The physical function recovery promotor of claim 1,
wherein
the stem cells are ES cells, EC cells, EG cells, iPS cells, hematopoietic stem cells, mesenchymal stem cells, hepatic stem cells, splenic stem cells, skin stem cells, umbilical cord blood stem cells, bone marrow stem cells, muscle stem cells, germ stem cells, fat stem cells, dental pulp stem cells, or MUSE cells.

3. The physical function recovery promotor of claim 1,
wherein
the reduced physical function is caused by an ischemic disease.

4. The physical function recovery promotor of claim 3,
wherein
the ischemic disease is any selected from myocardial infarction, unstable angina, graft occlusion after coronary artery bypass surgery, coronary artery occlusion after percutaneous transluminal coronary angioplasty, vascular occlusion after revascularization, arteriosclerosis obliterans, thromboangiitis obliterans, essential thrombocythemia, thrombotic thrombocytopenic purpura, antiphospholipid antibody syndrome, and Kawasaki disease.

5. The physical function recovery promotor of claim 3,
wherein
the ischemic disease is cerebral infarction.

6. The physical function recovery promotor of claim 3,
wherein
the ischemic disease is cerebral palsy.

7. The physical function recovery promotor of claim 6,
wherein
the cerebral palsy is caused by neonatal hypoxic-ischemic encephalopathy.

8. The physical function recovery promotor of claim 1,
wherein
the reduced physical function is caused by a sequela of cerebral infarction.

9. The physical function recovery promotor of claim 8,
wherein
the bone marrow mononuclear cells are intravenously administered.

10. The physical function recovery promotor of claim 8,
wherein
the CD34-positive cells are administered into a common carotid artery or vertebral artery.

11. The physical function recovery promotor of claim 8,
wherein
the CD133-positive cells are administered into a common carotid artery or vertebral artery.

12. The physical function recovery promotor of claim 1,
wherein
the reduced physical function is caused by an intractable nervous disease.

13. The physical function recovery promotor of claim 12,
wherein
the intractable nervous disease is any of amyotrophic lateral sclerosis (ALS), Parkinson's disease, multiple system atrophy, spinocerebellar ataxia, progressive supranuclear palsy, multiple sclerosis, syringomyelia, Guillain-Barre syndrome, spinal muscular atrophy, or myasthenia gravis.

14. The physical function recovery promotor of claim 1,
wherein
the reduced physical function is caused by dementia.

15. The physical function recovery promotor of claim 14,
wherein
the dementia is Alzheimer's disease dementia, dementia with Lewy bodies, frontotemporal dementia, corticobasal degeneration, cerebrovascular dementia, Down syndrome, or Huntington's disease.

16. The physical function recovery promotor of claim 1,
wherein
the reduced physical function is caused by autism.

17. The physical function recovery promotor of claim 1,
wherein
the reduced physical function is caused by developmental disability or behavioral disorder.

18. The physical function recovery promotor of claim 1,
wherein
the reduced physical function is caused by spinal cord injury.

19. The physical function recovery promotor of any one of claims 1-18,
wherein
the subject is an older person.

20. The physical function recovery promotor of any one of claims 1-19,
wherein
the physical exercise is performed using an assistive movement device, and
the assistive movement device includes:
an assistive movement attachment having an actuator configured to provide power to the subject;
a biological signal sensor configured to detect a biological signal of the subj ect;
a biological signal processing unit configured to obtain a neurotransmission signal and a muscle potential signal of the subject from the biological signal detected by the biological signal sensor;
a voluntary control unit configured to generate a command signal for causing the actuator to generate power according to the subject's intention, using the neurotransmission signal and the muscle potential signal obtained by the biological signal processing unit; and
a drive current generation unit configured to generate a current corresponding to the neurotransmission signal and a current corresponding to the muscle potential signal, based on the command signal generated by the voluntary control unit, and supply the currents to the actuator.

21. The physical function recovery promotor of claim 20,
wherein
the assistive movement device measures the subject's movement intention, and performs feedback adjustment while correcting a deviation of an actual movement from an ideal movement, while the subject senses the deviation of the actual movement from the ideal movement, and performs feedback adjustment of movement so as to minimize a variation in the deviation.

22. A physical function recovery promotor for promoting an effect of physical exercise for restoring a reduced physical function of a subject caused by a sequela of cerebral infarction, the physical function including a motor function, a cognitive function, and/or a sensory function, the promotor comprising:
bone marrow mononuclear cells.

23. The physical function recovery promotor of claim 22,
wherein
the bone marrow mononuclear cells are intravenously administered.

24. The physical function recovery promotor of claim 22 or 23,
wherein
the physical exercise is rehabilitation.

25. The physical function recovery promotor of any one of claims 22-24,
wherein
the physical function recovery promotor is administered to the subject during a period of time from 4 weeks before start of the physical exercise until start of the physical exercise.

26. The physical function recovery promotor of any one of claims 22-25,
wherein
the cerebral infarction is any of lacunar infarction, atherothrombosis cerebral infarction, or cardiogenic brain embolism syndrome.

27. A physical function recovery promotor for promoting an effect of physical exercise for restoring a reduced physical function of a subject caused by a sequela of cerebral infarction, the physical function including a motor function, a cognitive function, and/or a sensory function, the promotor comprising:
CD34-positive cells.

28. The physical function recovery promotor of claim 27,
wherein
the CD34-positive cells are administered into a common carotid artery or vertebral artery.

29. The physical function recovery promotor of claim 27 or 28,
wherein
the physical exercise is rehabilitation.

30. The physical function recovery promotor of any one of claims 27-29,
wherein
the CD34-positive cells are human umbilical cord blood-derived cells, G-CSF-mobilized peripheral blood CD34-positive cells, or human bone marrow-derived cells.

31. The physical function recovery promotor of any one of claims 27-30,
wherein
the physical function recovery promotor is administered to the subject during a period of time from 4 weeks before start of the physical exercise until start of the physical exercise.

32. The physical function recovery promotor of any one of claims 27-31,
wherein
the cerebral infarction is any of lacunar infarction, atherothrombosis cerebral infarction, or cardiogenic brain embolism syndrome.
